Europäisches Patentamt

European Patent Office

Office européen des brevets

(19)

(11) Publication number: **0 099 846**

**A1**

(12)

# EUROPEAN PATENT APPLICATION

(21) Application number: **83630084.8**

(22) Date of filing: **18.05.83**

(51) Int. Cl.³: **A 41 B 13/02**

(30) Priority: **20.07.82 GR 68802**
**14.10.82 GR 69536**

(43) Date of publication of application:
**01.02.84 Bulletin 84/5**

(84) Designated Contracting States:
**AT BE CH DE FR GB IT LI LU NL SE**

(71) Applicant: **Zervoudakis, John**
**4, Neophytou Vamva St.**
**Athens(GR)**

(72) Inventor: **Zervoudakis, John**
**4, Neophytou Vamva St.**
**Athens(GR)**

(74) Representative: **Schmitz, Jean-Marie et al,**
**Office Dennemeyer S.à.r.l. 21-25 Allée Scheffer P.O. Box**
**41**
**Luxembourg(LU)**

(54) **Improvements in diaper fastener tabs and tape stock for diaper fastener tabs.**

(57) A diaper tab and a diaper tab tape stock comprising a web construction made up of flexible first and second substrates. Each substrate has a fastening means on a first side. A release means is disposed on a second side of the second substrate. The first substrate (14) is substantially twice the thickness of the second substrate (24). The fastening means of the first substrate (14) is connected with the fastening means of the second substrate (24) along substantially a center line of the tape (10) to form a joining region. The second substrate (14) is folded back on itself over the joining region and over the first substrate (14) so that the release coating of the second substrate (24) contacts the fastening means of the first substrate (14). The edges of the first substrate (14) are substantially twice the thickness of the first substrate (14) so that the edges of the tape (10), which become the ends of the diaper tab, and the area in which the second substrate (24) is folded back on itself are all of substantially the same thickness. When the tape (10) is wound in layers on a roll sideward creep is avoided, thereby increasing the precision of placement of the diaper tap (10) on a diaper and greatly reducing the loss of finished diapers due to improper positioning of the tabs (10) on the diapers.

Fig. 1

EP 0 099 846 A1

## IMPROVEMENTS IN DIAPER FASTENER TABS AND TAPE STOCK FOR DIAPER FASTENER TABS

### Background of the Invention

### Field of the Invention

This invention relates to the field of fasteners used on disposable diapers. More particularly it relates to fastener tabs formed in a diaper producing machine from a diaper fastener tab tape stock and to a construction for the diaper fastener tab tape stock that provides significant manufacturing advantages.

### Description of the Prior Art

Prior art diaper tabs are generally constructed according to two major principles. The "single diaper tape" is a web construction of a unitary layer having uniform thickness along the tab (the machine direction) and consisting of two substrates bearing on each side adhesive and/or release means. The two substrate construction provides diaper tabs of the Y configuration with two arms being adapted to be fastened by the diaper manufacturer to both sides of one part of a diaper and the stem being adapted to be fastened to another part of the diaper by the user. The diaper is fastened by peeling back the stem which is folded over one of the two arms.

This diaper tab stock is supplied to the diaper manufacters as a single roll of tape which can be separated by the manufacturer into individual diaper tabs and applied to individual diapers. While the use of a single roll offers the advantage of relative simplicity, this type of diaper tab has the disadvantage that the construction is weak at the point where the two arms and the stem of the Y configuration meet. As a result it is possible, during the above mentioned peeling, for the stem to be completely separated or snapped from the two arms of the Y configuration diaper tab. This disadvantage is serious because, if this separation does occur, the diaper tab can no longer be used for joining the two parts of the diaper. This type of diaper tab also has the disadvantage of high cost due to the use of larger quantities of construction materials because it is constructed of two substrates which are equal in thickness in order for the diaper tab stock to have uniform thickness so that it can be conveniently wound on a spool.

A second type of diaper tab, the "two tape system" also has a "Y" configuration but one of the two arms carries an extenstion which is adhesively connected to the stem of the Y so that the risk of snapping, present in the previously described diaper tab type, is avoided. The diaper tab stock is supplied to the diaper manufacturers in two independent rolls which are separately placed on the diaper manufacturing machine, suitably combined to form the Y shape, separated into individual diaper tabs and applied to individual diapers. The diaper tab stock is supplied in two independent rolls becuase the Y construction is not of uniform thickness across the machine direction and cannot be rolled into a regular single roll usable by the diaper manufacturers. In addition, the formation of the Y shape by the diaper manufacturing line requires a decrease of the line's productivity of up to fifteen percent, skilled operators to oversee the manufacturing machine line and results in an increase in the rejection of finished diapers by up to ten percent because of improperly formed diaper tabs. Even though the diaper tab represents only approximately two percent of the cost of the diaper, the entire diaper is lost because the diaper tabs can be checked only after they have been applied to the diaper.

Summary of the Invention

The present invention provides a diaper tab tape stock which can be rolled on a core in layers over itself without creeping from side to side on the roll. As a result the precision of the positioning of the diaper tab on the diaper when the the tape is cut into tabs and applied to the diaper during the manufacturing process by the diaper machine is greatly enhanced and the rejection rate of finished diapers is significantly reduced to the point at which losses due to improperly placed tabs are virtually eliminated. In addition down time of a diaper manufacturing machine, which can produce up to 300 diapers per minute is reduced.

According to the present invention the diaper tab or a diaper tab tape stock, which is usable for forming diaper tabs, comprises a web construction made up of flexible first and second substrates. Each substrate has a fastening means on one side. A release means is disposed on the other side of the second substrate. The first substrate is substantially twice the thickness of the second substrate. The fastening means of the first substrate is in contact with the fastening means of the second substrate along substantially a center line of the tape to form a joining region. The second substrate is folded back on itself over the joining region and over the first substrate so that the release coating of the second substrate contacts the fastening means of the first substate. A release coating is also applied to the second surface of the first substrate.

The diaper tab further comprises means for increasing the thickness of the first substrate along each edge of the tape to define two regions having substantially twice the thickness of the first substrate. These thickened regions may simply constitute the first substrate being folded back on itself, the fold being secured by the adhesive layer of the first substrate.

Thus the present invention provides a diaper tab tape stock which has three regions which are of equal thickness across its width. Two of these regions are the folds at the edges of the first substrate while the third region is formed by the thickness of the first substrate and the second substrate being folded back upon itself. Thus, these three regions all have a thickness equal to substantially twice the thickness of the first substrate. It is this structure that prevents creep from side to side when the tape is rolled in layers on a core. Thus in a typical diaper manufacturing machine, when a diaper tab tape stock according to the present invention is used, only two tapes are needed, one being cut up into tabs for each side of the diapers. This greatly simplifies the manufacturing operation by eliminating the need for two rolls for each side of the diaper.

In addition, the present invention results in substantial savings in material over prior art double layer composite tapes due to the decreased width and thickness of the second substrate.

While the invention contemplate the use of two substrates the second substrate may also be comprised of two separate and coplanar substrates.

## BRIEF DESCRIPTION OF THE DRAWINGS

For the purposes of illustrating the invention, there are shown in the drawings embodiments which are presently preferred it being understood, however, that the invention is not limited to the precise arrangements and instrumentalities shown.

FIG. 1 is a cross sectional view of a diaper tab or a diaper tab tape stock according to the invention.

FIG. 2 is a cross sectional view of a diaper tab according to the invention applied to a first part of a diaper.

FIG. 3 is a cross sectional view which illustrates the manner in which the stem of the diaper tab of FIG. 2 is peeled back from one of the arms of the diaper tab in preparation for fastening to a second part of the diaper.

FIG. 4 is a cross sectional view showing the diaper tab of FIG. 2 fastened to a second part of the diaper.

FIG. 5 is a perpective view of a portion of a diaper tab tape stock according to the invention.

FIG. 6 is a cross sectional view illustrating an alternate embodiment of the invention.

## DETAILED DESCRIPTION OF THE PREFERRED EMBODIMENTS

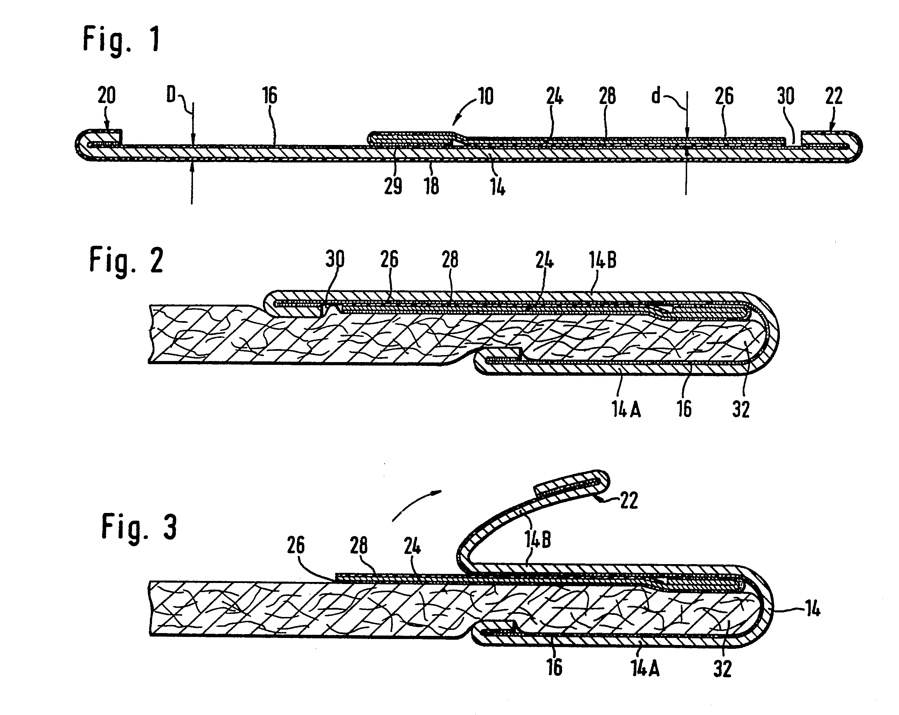

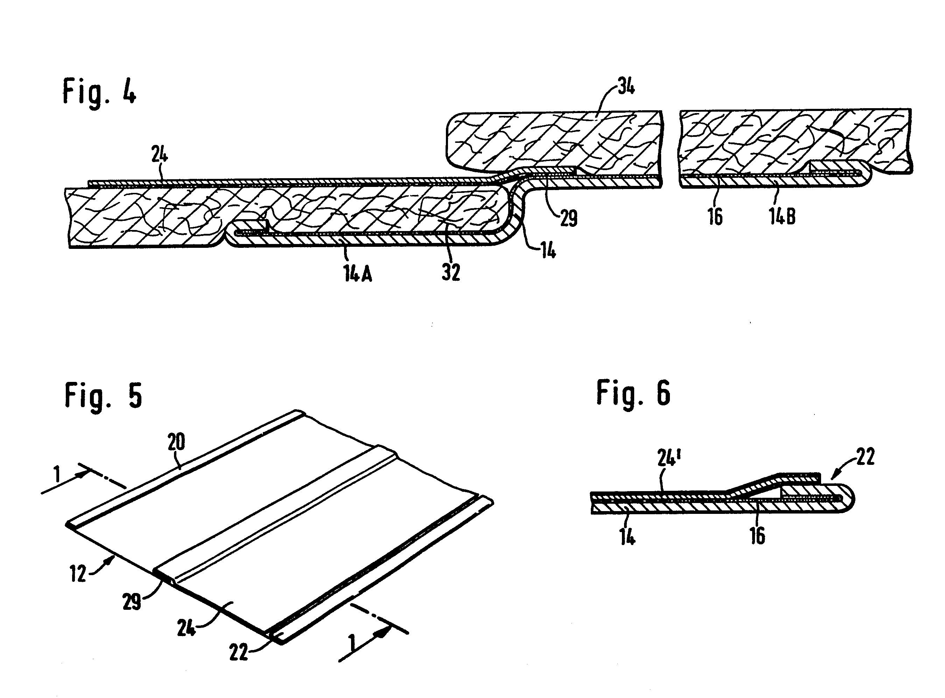

Referring now to the drawings wherein like numerals indicate like elements, there is shown in Fig. 1 a cross-section through a diaper tape according to the invention. Fig. 1 also represents a cross-section of the diaper tab tape stock of FIG. 5 taken along line 1-1 of FIG. 5. A diaper tab 10 is formed by cutting the tape 12 of FIG. 5, at fixed intervals along spaced apart parallel lines running in the direction of line 1-1 in a diaper manufacturing machine immediately prior to application of the diaper tab 10 to a first part of the diaper as illustrated in FIG. 2.

Diaper tab 10 is comprised of a first substrate 14 which defines the length of the tab in Fig. 1 and the width of tape 12 in Fig. 5. The base material for the substrate 14 may be one of a tradition material such as paper or plastic film. The plastic film may be composed of polyethylene, polypropylene, polyester or any any similar material. Alternatively laminated materials or saturated materials such as those saturated with an emulsion of rubber may be used. Another possibility is a metallic foil. These well known materials are chosen for being water proof, for their resistance to deterioration when exposed to waste products such as urine and for their mechanical strength.

An adhesive layer 16 is deposited on a first surface of substrate 14. Adhesive layer 16 may be solvent based, water based or a hot melt material all of which are well known in the art.

The surface of substrate 14 opposite to the surface on which the adhesive layer 16 is applied is coated with a release agent 18. Release agent 18 may be any of several well known silicone release agents.

The ends of substrate 14 are bent back on one another to form folds 20 and 22 which are secured due to the action of adhesive layer 16. Thus, two edge regions each having a thickness of substantially twice the thickness of substrate 14 are formed at the ends of the tab, or with reference to FIG. 5, along the edges of the tape stock.

A second substrate 24 has a thickness represented by the letter "d" equal to substantially one half the thickness of substrate 14 as represented by the letter "D". It is also comprised of one of the well known substrate materials referred to above with respect to substrate 14. It is preferrably chosen, however, so that its strength is substantially equal to the strength of substrate 14 despite the fact that it is half the thickness thereof. A first surface of substrate 24 is coated with an adhesive layer 26 similar in nature to adhesive layer 16 referred to above. The surface of substrate 24 opposite the one coated with adhesive layer 26 is coated with a release agent 28, similar to release agent 18.

Substrate 24 is joined to substrate 14 by means of the action between adhesive layer 16 and adhesive layer 26 at a joining region 29. Region 29 is at substantially the center of the length of diaper tab 10 and extends approximately along the center line of tape 12 as shown in FIG. 5.

Substrate 24 is folded over itself over region 29 and extends across substrate 14 toward fold 22.

Thus the diaper tab or diaper tab tape stock of Fig. 1 has three regions which are of virtually identical thickness. These are the regions containing folds 20 and 22 and the area of joining region 29. As noted above the presence of these three regions of virtually identical thickness minimizes sideward creep when the tape 12 is wound in layers on a roll.

In the embodiment shown in FIG. 1 substrate 24 does not extend as far as fold 22. Thus, there is a gap 30. Adhesive layer 16 is exposed by virtue of gap 30.

Figure 2 illustrates the manner in which the diaper tab of FIG. 1 is applied to a diaper by a diaper manufacturing machine. A first portion 14A of substrate 14 contacts a portion of a first side of a first part 32 of the diaper and is secured thereto by means of the action of adhesive layer 16 of substrate 14. The combination of substrate 24 and second portion 14B of substrate 14 is forced into contact by appropriate portions of the diaper manufacturing machine with a portion of the second side of the first part of the diaper opposite the portion of the first side of the first part of the diaper to which portion 14A is applied and is secured thereto by means of the action of adhesive layer 26 of substrate 24. Thus, figure 2 also illustrates the manner in which the diaper tab is positioned on the diaper by the diaper manufacturing machined prior to use to use by the consumer. As noted above, adhesive layer 16 remains exposed and is free to come in contact with the second surface of the diaper through gap 30. This provides a small amount of adhesion which serves to secure portion 14B to the second side of the diaper. This adhesion is not so great, however, that it can not easily be overcome in order to peel portion 14B of substrate 14 away from substrate 24 which remains in contact with the second surface of the diaper as may be more readily appreciated from FIG. 3.

As shown in FIG. 3 portion 14B of substrate 14 is readily peeled away from substrate 24 due to the presence of release agent 28. Fold 22, the external surface of which is coated only with a release coating, forms a handle which may be readily gripped to peel portion 14B of substrate 14 back from substrate 24.

While portions 14A and 14B of substrate 14 are comprised of the same member and are therefore integral with one another, it should be recognized that this is merely the preferred form of the invention. Portion 14A and portion 14B could conceivably be two different members that would, according to the invention, be of the same thickness and would be coplanar with one another when configured in a manner similar to the diaper tab tape stock shown in Fig. 5.

In Fig. 4 portion 14B of substrate 14 has been completely peeled away from substrate 24, thus unfolding substrate 24 as well. While portion 14A of substrate 14 and substrate 24 form two arms of the diaper tab which are spaced apart by the first part 32 of the diaper, portion 14B of substrate 14 forms a third part or substrate which may be fastened due to the action of adhesive layer 16 to a second part 34 of the diaper. Such fastening serves to secured the diaper in place on the user.

While the joining region 29 is shown as being substantially in the same plane as the second side of the first part of the diaper (the surface in contact with substrate 24), this is by no means essential. In fact the diaper tab may be positioned on the diaper by the diaper manufacturing machine so that joining region 29 is disposed half way between the first surface and the second surface of the first part 32 of the diaper, or closer to the first surface, as may be required.

In the embodiment of the invention illustrated in FIG. 6 the second substrate 24' extends over fold 22. Thus there is no gap exposing adhesive layer 16. In all other respects the construction of the diaper tab according to FIG. 6 and the diaper tab tape stock from which the tab is formed, is identical to that described above. In the embodiment of FIG. 6, however, the ultimate thickness of the diaper tab in the region of overlap of substrate 24' and fold 22 is approximately twenty five percent thicker than the thickness of fold 22 alone. This construction is advantageous in certain applications. The additional thickness caused by the overlap of substrate 24' and fold 22 does not significantly affect the ability of the tape to resist creeping from side to side when wound in layers on a roll.

In addition to the advantage of reducing creep when a diaper tape according to the present invention is layered on a roll, as noted above the present invention provides for a smaller quantity of material to be utilized for the construction of the diaper tab as compared to prior art constructions. A ten to twenty percent savings in material can be achieved depending on the required length of the diaper tab. This savings in material is best illustrated in the example below:

EXAMPLE

A diaper tab is constructed according to FIG. 1. The first substrate 14 has a total length of 66 millimeters before the ends are folded over to form folds 20 and 22 which may each be anywhere from three to six millimeters across. Substrate 24 has a length of 38 millimeters. Substrate 14 and 24, initially stored on separate rolls, are rolled together to form joining region 29 according to a process and with apparatus that is well known in the art. Where a gap 30 is utilized, it has a dimension of between one and two millimeters, thus exposing a strip of adhesive layer 16 of this width. The diaper tab tape stock according to the invention is cut along its length approximately every 20 millimeters by the diaper forming machine to form each diaper tab.

The total width of material utilized according to the above example, equal to the sum of the widths of the first and second substrates, is approximately 104 millimeters. A double layer composite tape similar to one constructed according to the prior art would typically use a material width of 62 millimeters for the first substrate and 62 millimeters for the second substrate resulting in a total material width of 124 millimeters. Thus, a savings of approximately 16 percent is achieved on the basis of the above example. A further savings is achieved because one of these substrates according to the present invention is of reduced thickness thus further decreasing the amount of material required.

The present invention may be embodied in other specific forms without departing from the spirit or essential attributes thereof and accordingly reference should be made to the appended claims rather than to be foregoing specification as indicating the scope of the invention.

What is claimed is:

1.      A diaper tab tape stock comprising:

a web construction made up of flexible first and second substrates, each substrate having a fastening means on a first side thereof, and the second substrate having a release means on a second side thereof;

the first substrate being substantially twice the thickness of the second substrate, the fastening means of the first substrate being in contact with the fastening means of the second substrate along substantially a center line of the tape to form a joining region;

the second substrate being folded back on itself over the joining region and over the first substrate so that the release coating of the second substrate contacts the fastening means of the first substrate.

2.      The diaper tab tape stock of claim 1, in which the first substrate has a release means on a second side thereof.

3.      The diaper tab tape stock of claim 1 in which the second substrate extends to a point short of the edge of the first substrate.

4.      The diaper tab tape stock of claim 1 further comprising means for increasing the thickness of the first substrate along each edge of the tape, to define two regions having substantially twice the thickness of the first substrate.

5.      The diaper tab tape stock of claim 1 in which the second substrate extends over the fastening means of the first substrate to a point short of an edge of the first substrate, whereby a portion of the fastening means of the first substrate is left exposed between an end of the second substrate and the edge.

6.      The diaper tab tape stock of claim 1 in which the second substrate extends over the first fold of the first substrate.

7.      A diaper tab tape stock comprising:

a first substrate defining the width of the tape and having a first thickness;

a first means for fastening the first substrate to a diaper, the first means being disposed on a surface of the first substrate;

a first fold having a thickness equal to substantially twice the thickness of the first substrate, the first fold being at a first edge of the first substrate, the first fold comprising a part of the first substrate folded back upon itself so that the first means secures the first fold;

a second fold having a thickness equal to substantially twice the thickness of the first substrate, the second fold being at a second edge of the first substrate, the second fold comprising a part of the first substrate folded back upon itself so that the first means secures the second fold;

a second substrate having a second thickness, the second thickness being equal to substantially one half the thickness of the first substrate;

a second means for fastening the second substrate to a diaper, the second means being disposed on a first surface of the second substrate;

a release means disposed on a second surface of the second substrate; the second surface being opposite the first surface;

a joining region at which the first substrate and the second substrate are joined along substantially a center line of the first substrate;

the second substrate being folded back on itself over the joining region and extending over the first substrate with the release means being in contact with the first means.

8.      The diaper tab tape stock of claim 7 in which the second substrate extends across the first substrate to a point short of the first fold so that a portion of the first means is left exposed between an edge of the second substrate and the first fold.

9.      The diaper tab tape stock of claim 7 in which the second substrate extends across the first substrate so as to partially cover the first fold of the first substrate.

10.      The diaper tab tape stock of claim 7 in which the second substrate has substantially the same strength as the first substrate.

11.     The diaper tab tape stock of claim 7 in which the first substrate and the second substrate are joined as a result of the first means and the second means being in contact along a portion of the width of the tape to define the joining region.

12.     A diaper tab for fastening diapers, the diaper tab comprising:

a web construction made up of flexible first and second substrates, each substrate having a fastening means on a first side thereof, and the second substrate having a release means on a second side thereof;

the first substrate being substantially twice the thickness of the second substrate, the fastening means of the first substrate being in contact with the fastening means of the second substrate along substantially a center line of the tape to form a joining region;

the second substrate being folded back on itself over the joining region and over the first substrate so that the release coating of the second substrate contacts the fastening means of the first substrate.

13.     The diaper tab of claim 12 in which the first substrate has a release means on a second side thereof.

14.     The diaper tab of claim 12 in which the second substrate extends to a point short of an edge of the first substrate.

15.     The diaper tab of claim 12 in which the first substrate is folded back on itself along each edge of the tape to form a fold along each edge, the fastening means serving to secure the folds, the folds being regions having substantially twice the thickness of the first substrate.

16.     The diaper tab of claim 15 in which the second substrate extends to over the fastening means of the first substrate to a point short of a first of the folds, whereby a portion of the fastening means of the first substrate is left exposed between an end of the second substrate and the first fold.

17.     The diaper tab of claim 14 in which the second substrate extends over the first fold of the first substrate.

18.      A fastener tab for a disposable diaper, the fastener tabe comprising:

a first substrate adapted to be fastened to a first side of a first part of a diaper;

a first means for fastening the first substrate to the first side;

a second substrate adapted to be fastened to a second side of the first part of the diaper so that the first substrate and the second substrate are facing one another and spaced apart by the thickness of the diaper, the second substrate having a thickness substantially half that of the first substrate;

a second means for fastening the second substrate to the second side of the first part of the diaper;

a joining region, the first substrate and the second substrate being fastened to one another at the joining region;

a third substrate attached to the joining region, the third substrate being for attachment to a second part of the diaper so that the diaper may be fastened on a wearer, the third portion having a thickness substantially equal to that of the first portion;

a third means for fastening the third substrate to the second part of the diaper.

19.      The fastener tab of claim 18 in which the second substrate is folded back on itself over the joining region, the second substrate having a release coating on a surface thereof opposite to a surface on which the second means is disposed, the second substrate extending with the release coating in contact with the third means.

20.      The fastener tab of claim 18 in which the first substrate is folded back upon itself at an edge thereof so that the first means secures a portion of the first substrate to form a first fold having a thickness equal to substantially twice the thickness of the first substrate.

21.      The fastener tab of claim 18 in which the third substrate is folded back upon itself at an edge thereof so that the third means secures a portion of the third substrate to form a second fold having a thickness equal to substantially twice the thickness of the third substrate.

0099846

-14-

22.      A diaper tab tape stock usable for forming adhesive diaper tabs, the diaper tab tape stock comprising:

a first substrate, having a first thickness;

a first means for fastening the first substrate to a diaper, the first means being disposed on a surface of the first substrate;

the first substrate being folded back upon itself at an edge thereof so that the first means secures a portion of the first substrate to form a first fold having a thickness equal to substantially twice the thickness of the first substrate;

a second substrate having a second thickness, the second thickness being equal to substantially one half the thickness of the first substrate;

a second means for fastening the second substrate to a diaper, the second means being disposed on a first surface of the second substrate;

a release means disposed on a second surface of the second substrate;

a joining region at which the first substrate and the second substrate are joined as a result of the first means and the second means being in contact with one another along a portion of the width of the tape;

a third substrate attached to the joining region, the third substrate having a thickness substantially equal to the thickness of the first substrate, the third substrate being substantially coplanar with the first substrate;

a third means adapted for fastening the third substrate to a diaper;

the second substrate being folded back on itself over the joining region and extending over the third substrate with the release means being in contact with the third means;

the third substrate being folded back upon itself at an edge thereof so that the third means secures a portion of the third substrate to form a second fold having a thickness equal to substantially twice the thickness of the third substrate.

Fig. 1

Fig. 2

Fig. 3

1 / 2

0099846

Fig. 4

34

24

29

16    14B

14A    32    14

Fig. 5

20

1

12

29

24

22

1

Fig. 6

24'    22

14    16

2 / 2

0099846

European Patent
Office

**EUROPEAN SEARCH REPORT**

Application number

| | | | | |
|---|---|---|---|---|
| **DOCUMENTS CONSIDERED TO BE RELEVANT** | | | EP 83630084.8 | |
| Category | Citation of document with indication. where appropriate, of relevant passages | Relevant to claim | CLASSIFICATION OF THE APPLICATION (Int. Cl. ³) | |
| A | GB - A - 1 593 710 (AVERY INTER-NATIONAL CORPORATION) <br> * Totality * <br> -- | 1,7,12 | A 41 B 13/02 | |
| A | US - A - 4 020 842 (REICHMAN et al.) <br> * Fig. 2-6 * <br> ---- | 1,7,12, 18,22 | | |
| | | | TECHNICAL FIELDS SEARCHED (Int. Cl. ³) | |
| | | | A 41 B | |
| The present search report has been drawn up for all claims | | | | |
| Place of search <br> VIENNA | Date of completion of the search <br> 31-08-1983 | | Examiner <br> NETZER | |